(19)

**Europäisches Patentamt**
**European Patent Office**
**Office européen des brevets**

(11) **EP 4 502 469 B1**

(12) **EUROPEAN PATENT SPECIFICATION**

(45) Date of publication and mention
of the grant of the patent:
**22.07.2026 Bulletin 2026/30**

(21) Application number: **24192259.0**

(22) Date of filing: **01.08.2024**

(51) International Patent Classification (IPC):
**F23N 5/08** (2006.01)

(52) Cooperative Patent Classification (CPC):
**F23N 5/082;** F23N 2229/04; F23N 2229/16;
F23N 2229/20

(54) **SINGLE-PIXEL MULTISPECTRAL IMAGER FOR FLARE AND BURNER COMBUSTION EFFICIENCY MEASUREMENT**

MULTISPEKTRALER EINZELPIXEL-BILDGEBER ZUR MESSUNG DER VERBRENNUNGSEFFIZIENZ EINER FACKEL UND EINES BRENNERS

IMAGEUR MULTISPECTRAL À PIXEL UNIQUE POUR MESURE DE L'EFFICACITÉ DE COMBUSTION D'UNE TORCHE ET D'UN BRÛLEUR

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **03.08.2023 US 202363517349 P**

(43) Date of publication of application:
**05.02.2025 Bulletin 2025/06**

(73) Proprietors:
• **Services Pétroliers Schlumberger**
**75007 Paris (FR)**
Designated Contracting States:
**FR**
• **Schlumberger Technology B.V.**
**2514 JG The Hague (NL)**
Designated Contracting States:
**AL AT BE BG CH CY CZ DE DK EE ES FI GB GR HR HU IE IS IT LI LT LU LV MC ME MK MT NL NO PL PT RO RS SE SI SK SM TR**

(72) Inventor: **CATHELINE, Sebastien**
**92143 Clamart (FR)**

(74) Representative: **Schlumberger Intellectual Property Department**
**Parkstraat 83**
**2514 JG Den Haag (NL)**

(56) References cited:
**WO-A1-02/070953          WO-A1-2021/085716**
**CN-A- 101 625 270      US-A1- 2001 035 952**
**US-A1- 2009 289 178    US-B2- 11 248 963**

# Description

## FIELD OF THE INVENTION

Aspects of the invention relate to imaging technology. More specifically, aspects of the invention relate to a multispectral imager for flare and burner combustion efficiency measurement.

## BACKGROUND

Industry continually attempts to become more efficient in operations to maximize profits and minimize risks. Use of energy has been a large focus for industry for decades. By efficiently using energy, the costs to manufacture products can be drastically reduced. As time progresses, energy costs generally rise, leading to a need for innovation.

Historically, burning of hydrocarbon fuels was accomplished through a few methods. In addition to burning of hydrocarbon fuels, waste products, such as waste gas streams, may contain hydrocarbons. Emission of raw hydrocarbon fuels to the atmosphere is strictly regulated; therefore, steps are undertaken to prevent such hydrocarbons from being emitted.

One method used to treat hydrocarbon gaseous waste streams is to use a flare system to burn the hydrocarbons, rather than emit them. Such systems can be problematic. Flares may need constant "tuning" where the quantity of hydrocarbons supplied to the flare is strictly regulated. If such streams are not regulated, incomplete combustion may occur, resulting in inefficient conversion of hydrocarbon to carbon dioxide. The flare system must be closely monitored such that the feed streams are not too rich or lean, providing an efficient burning process.

Several methods may be used to quantify the combustion efficiency of a flare. In some instances, qualified spotters are used to look at the emissions and visually determine if the gaseous emissions are being properly burned. In other, more automated systems, a series of cameras may be established to provide a visual view of the combustion. Still further embodiments monitor oxygen and fuel provided to the burner system to calculate the most efficient burning possible. In such systems, automatic controls are provided to an operations control room to adjust parameters, as needed. Document US 2001/0035952 describes a method for monitoring an optical system having a front lens disposed immediately at a combustion chamber. Document WO 02/070953 describes a measuring device for monitoring flames during a combustion process. Document WO 2021/085716 describes an apparatus and method for diagnosing combustion condition on basis of spontaneous flame emission spectroscopy. Document CN 101 625 270 describes a flame temperature field and combustion intermediate product concentration field monitoring system designed on basis of optical compensation.

As time has progressed, there is a greater need to become more efficient and provide for even tighter controls of flare or burner capabilities. The conventional aspects of burning technologies do not provide a sufficient measurement of combustion efficiency.

There is a need to provide an apparatus and methods that are easy to operate and that provide superior results compared to conventional apparatus and methods.

There is a further need to provide apparatus and methods that do not have the drawbacks discussed above, namely incomplete combustion and emissions of hydrocarbons to the environment.

There is a still further need to reduce economic costs associated with operations and apparatus described above with conventional tools.

## SUMMARY

So that the manner in which the above recited features of the present invention can be understood in detail, a more particular description of the disclosure, briefly summarized below, may be had by reference to embodiments, some of which are illustrated in the drawings. It is to be noted that the drawings illustrate only typical embodiments of this disclosure and are therefore not to be considered limiting of its scope, for the invention may admit to other equally effective embodiments without specific recitation. Accordingly, the following summary provides just a few aspects of the description and should not be used to limit the described embodiments to a single concept.

In one example embodiment, an apparatus for analyzing a combustion source is disclosed in claim 1.

In another example embodiment, a method for determining a combustion efficiency for a flare is disclosed in claim 13.

## BRIEF DESCRIPTION OF THE DRAWINGS

So that the manner in which the above recited features of the present invention can be understood in detail, a more particular description of the invention, briefly summarized above, may be had by reference to embodiments, some of which are illustrated in the drawings. It is to be noted; however, that the appended drawings illustrate only typical embodiments of this invention and are therefore not be considered limiting of its scope, for the invention may admit to other equally effective embodiments.

FIG. 1 is a side elevational view of a flaring system used in industry and a graph of combustion efficiency for the flaring system.

FIG. 2 is a graph of spectral bands of interest for a mid-infrared region of a combustion process being analyzed.

FIG. 3 is one example embodiment of an aspect of the invention with a transceiver that uses multiple detectors.

FIG. 4 is one example embodiment of an aspect of the invention with a transceiver using a diffraction grating and a detector array.

FIG. 5 is a side view of a prior art photovoltaic detector.

FIG. 6 is a diagram illustrating a dichroic filter principle.

FIG. 7 is a diagram of a Dichroic filter proposal in one example embodiment of the invention.

FIG. 8 illustrates the use of a Dichroic filter to isolate a hydrocarbon band.

FIG. 9 is an illustration of a Dichroic filter used to isolate a carbon dioxide band (around 4.26um from the reference band (around 3.9um).

FIG. 10 is a view of a dual wedge prism scanner.

FIG. 11 is a depiction of a fast-steering mirror scanner.

FIG. 12 is a method for performing an analysis of combustion efficiency measurement in one example embodiment of the invention.

To facilitate understanding, identical reference numerals have been used, where possible, to designate identical elements that are common to the FIG.s ("FIGS"). It is contemplated that elements disclosed in one embodiment may be beneficially utilized on other embodiments without specific recitation.

**DETAILED DESCRIPTION**

In the following, reference is made to embodiments of the invention. It should be understood; however, that the invention is not limited to specific described embodiments. Instead, any combination of the following features and elements, whether related to different embodiments or not, is contemplated to implement and practice the invention. Furthermore, although embodiments of the invention may achieve advantages over other possible solutions and/or over the prior art, whether or not a particular advantage is achieved by a given embodiment is not limiting of the invention. Thus, the following aspects, features, embodiments and advantages are merely illustrative and are not considered elements or limitations of the claims except where explicitly recited in a claim. Although the terms first, second, third, etc., may be used herein to describe various elements, components, regions, layers and/or sections, these elements, components, regions, layers and/or sections should not be limited by these terms. These terms may be only used to distinguish one element, components, region, layer or section from another region, layer or section. Terms such as "first", "second" and other numerical terms, when used herein, do not imply a sequence or order unless clearly indicated by the context. Thus, a first element, component, region, layer or section discussed herein could be termed a second element, component, region, layer or section without departing from the teachings of the example embodiments.

When an element or layer is referred to as being "on," "engaged to," "connected to," or "coupled to" another element or layer, it may be directly on, engaged, connected, coupled to the other element or layer, or interleaving elements or layers may be present. In contrast, when an element is referred to as being "directly on," "directly engaged to," "directly connected to," or "directly coupled to" another element or layer, there may be no interleaving elements or layers present. Other words used to describe the relationship between elements should be interpreted in a like fashion. As used herein, the term "and/or" includes any and all combinations of one or more of the associated listed terms.

Some embodiments will now be described with reference to the FIGs. Like elements in the various FIGs will be referenced with like numbers for consistency. In the following description, numerous details are set forth to provide an understanding of various embodiments and/or features. It will be understood, however, by those skilled in the art, that some embodiments may be practiced without many of these details, and that numerous variations or modifications from the described embodiments are possible. As used herein, the terms "above" and "below", "up" and "down", "upper" and "lower", "upwardly" and "downwardly", and other like terms indicating relative positions above or below a given point are used in this description to more clearly describe certain embodiments.

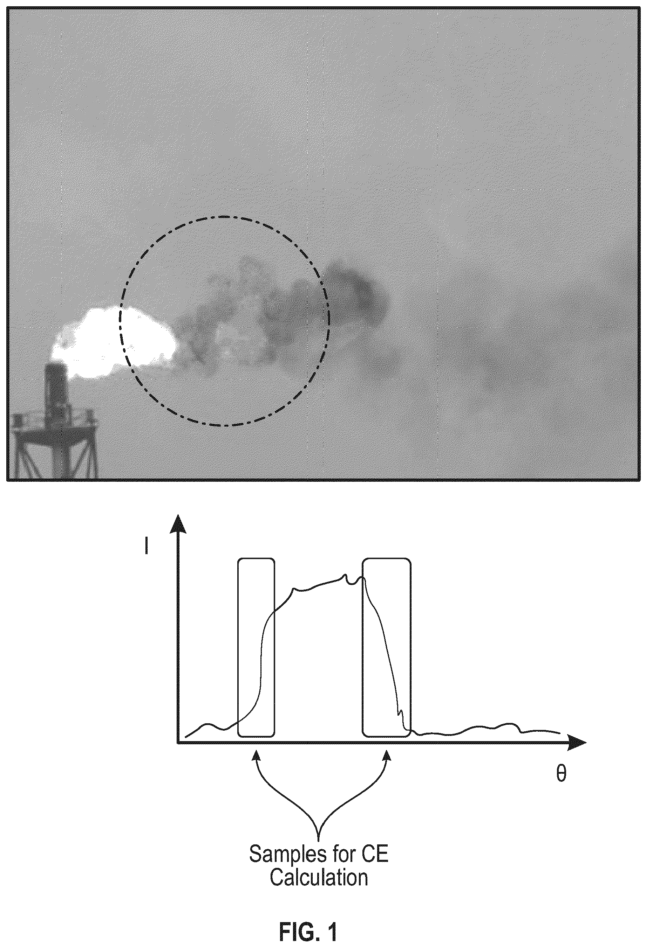

Referring to FIG. 1, aspects of the invention provide for a device to measure the combustion efficiency, hereinafter called "CE" of a gas flare or oil burner. At the top of FIG. 1, a flare side elevational view is provided. As illustrated, aspects of the disclosure may be where gas or oil is burned in the open atmosphere. Such non-limiting embodiments may include well test operations, oil refineries, production facilities and other petrochemical plants. Combustion efficiency is a quantity which characterizes the performance of the combustion where the goal is to convert the hydrocarbon to carbon dioxide, since it has a lower greenhouse effect than other gas hydrocarbons, such as methane. The quantity is defined as follows:

$$CE\ (\%) = 100\ \frac{[CO_2]}{[CO_2]+n.[UHC]+[CO]}\quad,$$

but there are several other ways to define it depending on which gas species are considered. The values with the brackets refer to the concentration in parts per million (ppm), UHC stands for unburned hydrocarbon, and n. is the average number of carbon molecules in the unburned hydrocarbon. As provided at the bottom of FIG. 1, a graph showing combustion efficiency calculations is presented.

Aspects of the invention provide for a device that uses an imager system in the mid infrared region, between 3 and 5 um which collects light from the flame to compute the combustion efficiency (CE). The hot combustion gases in the flame within the flare or burner emit infrared light in specific wavelength bands; so, it is possible to infer the relative gas concentration in the flame from the spectral content of this light. Aspects of the disclosure operate by first collecting light from different locations in and around the flame using a scanner (steered mirror, galvo scanner-a scanner in which one or two mirrors are tilted to deflect a beam, or dual wedge prism scanner).

In the aspects described, the collected light is then split into several streams depending on wavelength. The light is then filtered to target specific spectral bands of interest such as the hydrocarbon band between 3200nm and 3400nm, the carbon dioxide band around 4260nm, and a reference in between 3500 and 4000 nm. Other bands of interest can be added such as the carbon monoxide band around 4500 and 4800nm. The individual streams of light are sent to different detectors to produce a signal proportional to the intensity in the specific spectral band. These signals are then used to compute the ratio of gas species.

The idea behind this measurement is to scan an area where a flame is present and extract the samples from the boundary of the flame where the gases are still hot so they emit light but the combustion is finished. Focusing the analysis on the boundary of the flame ensures that the CE can be calculated reliably. In embodiments, scanning may be used to generate cross section of the flame to make the detection of the flame boundary as simple as possible.

In the aspects described here, a single-pixel photon multispectral imager is used, as this imager does not produce a multi-spectral image like conventional apparatus but rather a single pixel at each sampling time. Due to the use of a scanner to aim at different targets, it is possible to reconstruct a full image of a scene in the different spectral bands of interest. Other possibilities exist than described above.

The concept of the measurement is to collect the light from in and around the flame using a telescope coupled with a scanner. When the scanner aims at the flame, the light emitted by the hot combustion gas is received and analyzed in different spectral bands. The CE can be computed in the boundary of the flame where the combustion is finished, but the combustion gases are still hot enough to emit light. By scanning across the flame, a signal intensity is observed similar to the one shown in FIG. 1. A sample on the boundary layer may be used to compute the CE.

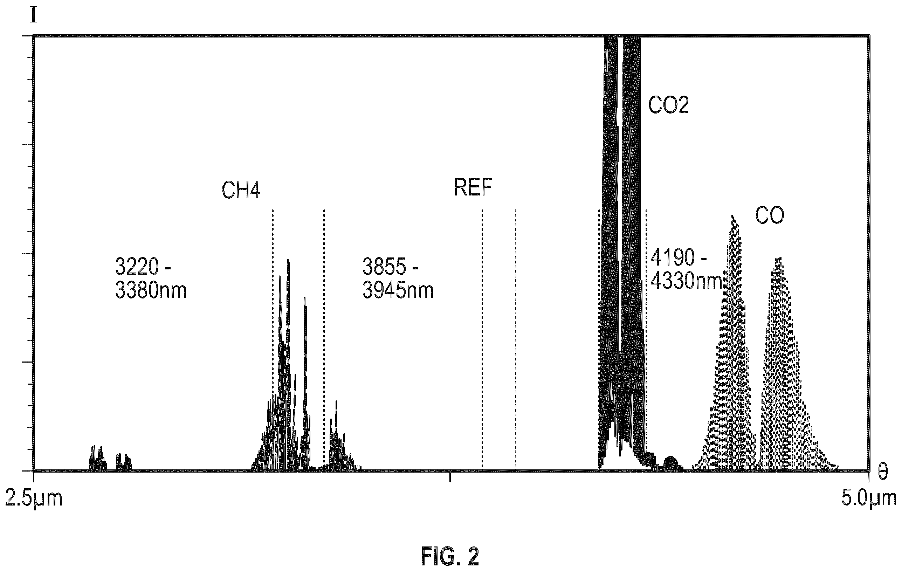

Referring to FIG. 2, the absorption coefficient of different gases of interest in the 2.5 to 5 um range is illustrated. For this measurement, we are interested mainly in the hydrocarbon band 3200-3400 nm, the carbon dioxide band 4190 to 4330 nm, a reference band somewhere between the hydrocarbon and carbon dioxide band, and if required the carbon monoxide band 4700-4800 nm. The idea of this measurement is to extract the intensity in each band of interest and compute the corresponding integrated concentration knowing the average absorption coefficient of each gas and the filters shape. Then it is possible to compute the CE. The reference band is used to correct the other intensities to remove broadband background radiation, such as the one coming from soot particles. As will be understood, other wavelength possibilities may be used than those described above.

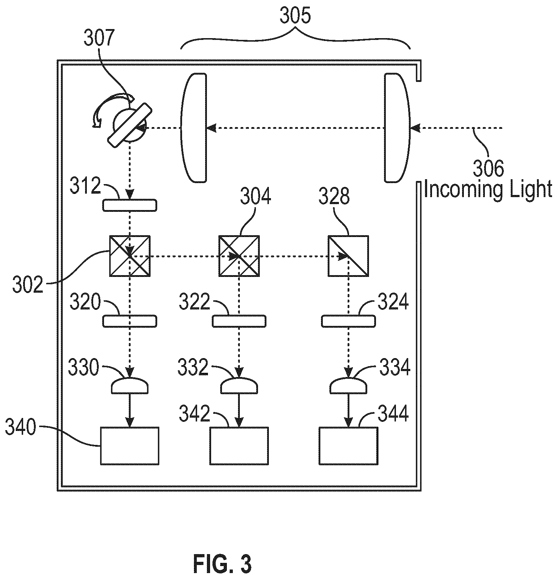

Referring to FIG. 3, an implementation of the device **300** is disclosed. Incoming light **306** is accepted by a telescope **305** and transmits the light to the scanner **307.**. As will be understood, various optical instruments may be used in conjunction with the device **300.** As disclosed, a telescope **305** may be used. Different types of lenses within the telescope **305** may be used for help in transmitting the light. A first broadband filter **312** removes unwanted wavelengths received. In one example embodiment, a 3 to 5 um bandpass filter may be used. A specific window may be used to reject the same wavelength. The light is then separated by two beam splitters **302, 304.** These beam splitters can be standard ones, meaning they are wavelength independent, or specific dichroic filters which will split the light based on the wavelength. The latter avoids wasting light which improves the signal-to-noise ratio. Each stream of light then goes through a specific "narrow" bandpass filter (100-200 nm width) targeting a specific species. The light in each stream is then focused onto a detector. Through use of the beam splitters **302, 304,** and mirror **326,** and associated narrowband filters **320, 322, 324,** and accompanying lenses **330, 332, 334,** the wavelengths impact on detectors **340, 342** and **344.**

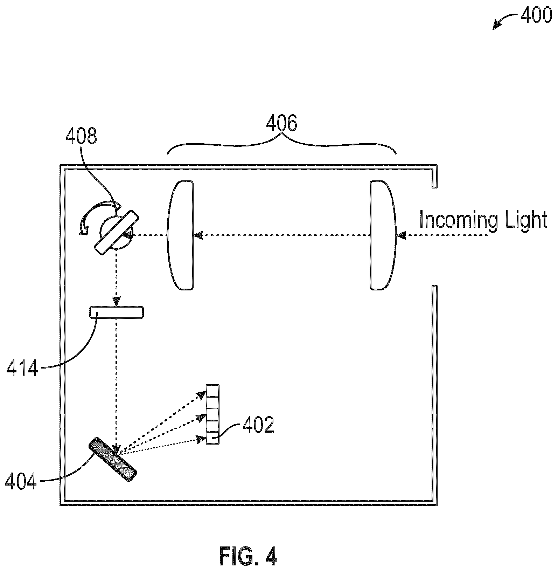

A second embodiment is illustrated in FIG. 4. In this embodiment **400**, a linear detector array **402** is used instead of a single detector coupled with a diffraction grating **404.** The light is diffracted by the grating based on its wavelength, which allows separation of the wavelength. The signal on each detector of the array allows computing a full spectrogram from which computations may be similarly performed for each gas. Light passes through a telescope **406** and is reflected off a scanner **408.** The light then passes through a broadband filter **414,** before bouncing off the diffraction grating **404** to the detector array **402.** In both embodiments disclosed, a housing may be created to contain the components except the first lens. Other configurations are possible and as such, should not be considered limiting. As will be understood and as previously disclosed, various optical instruments may be used in place of the telescope **406** disclosed. In other embodiments, multiple lenses may be used within the telescope.

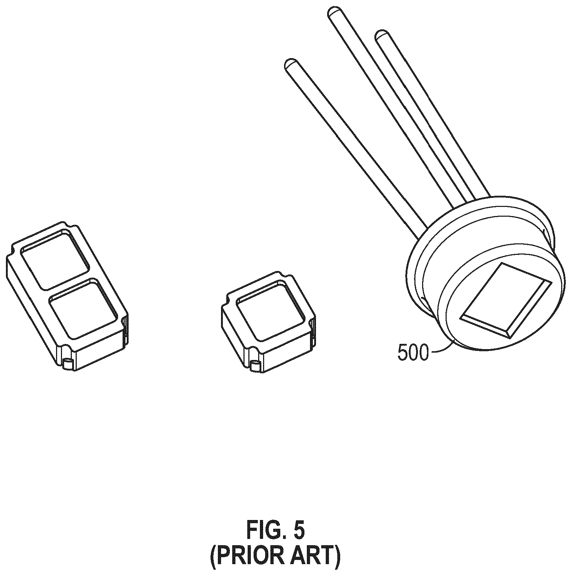

FIG. 5 shows a conventional photovoltaic detector **500** which can be used for this application because they have relatively high sensitivity, high linearity, and extremely high speed. In embodiments, a unit may be used because it includes filters for the hydrocarbon, carbon monoxide, and reference band. In addition, different types of detectors may be used, such as a non-cooled detector, which means it does not require a thermo-electric cooler. Both cooled and non-cooled detector may be used in different embodiments.

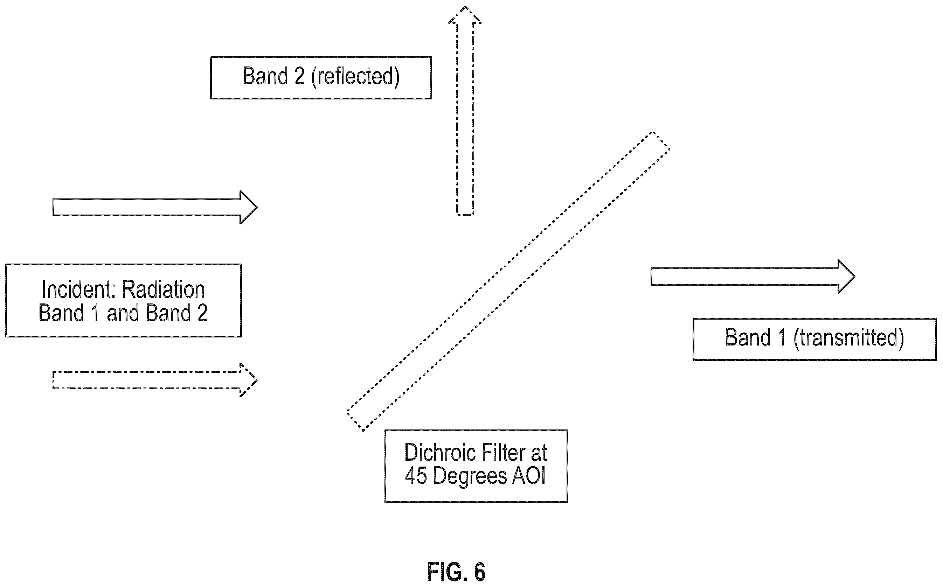

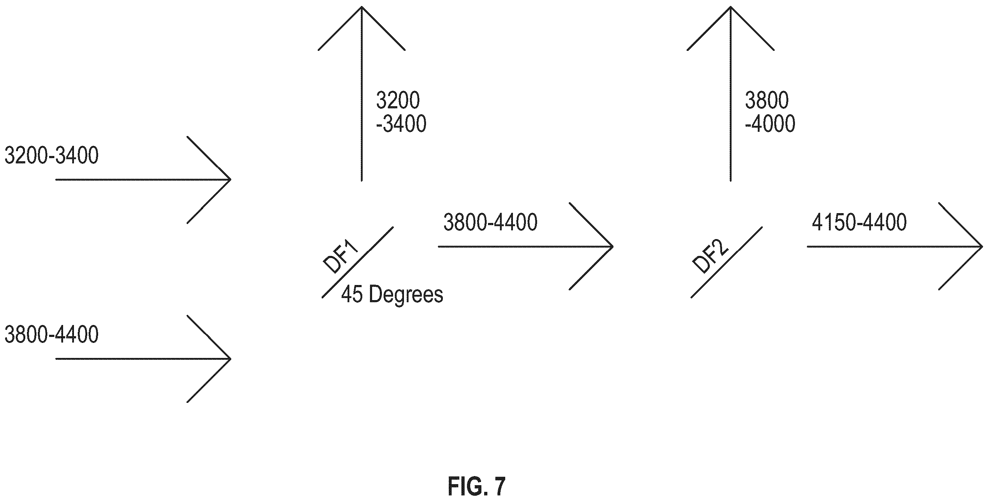

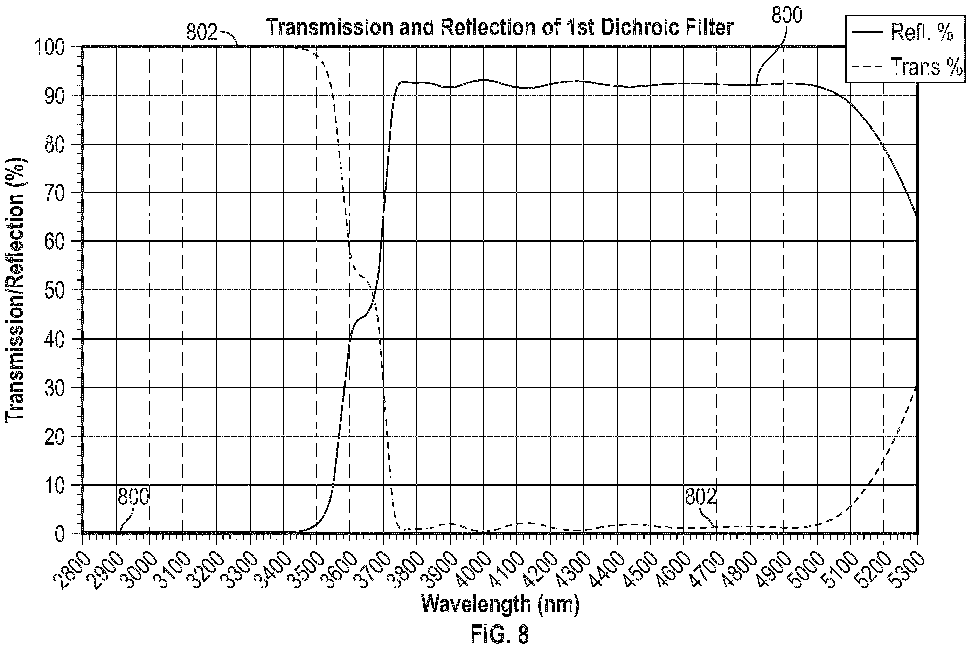

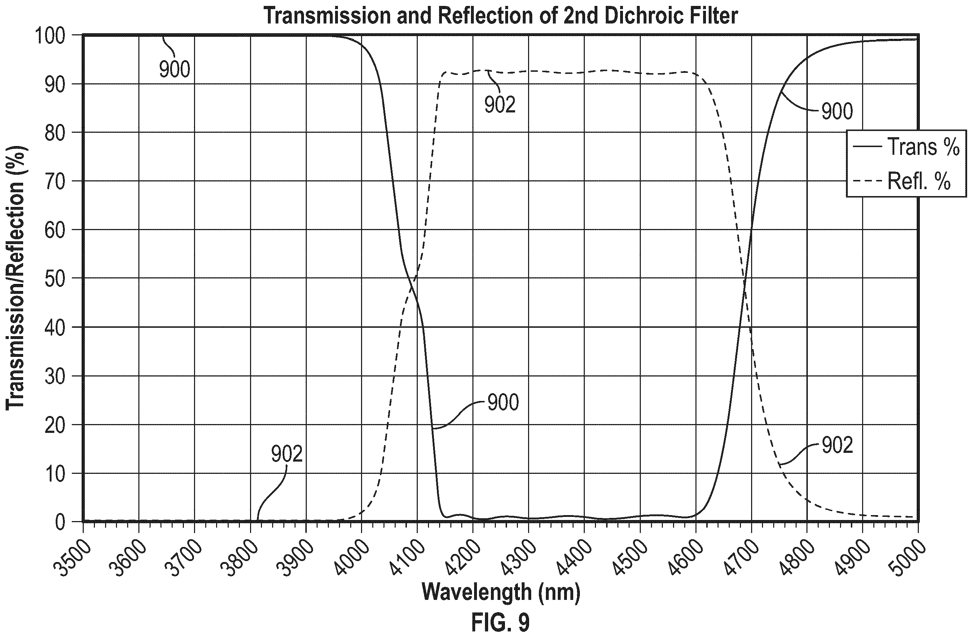

Referring to FIG. 6, arrangements using dichroic filters are disclosed. As previously discussed, the dichroic filters split the light between transmission and reflection depending on the wavelength. With these filters, all the light from the hydrocarbon and carbon dioxide bands may be extracted and sent to the detector with minimal losses. Sample detectors are shown in FIG. 7. The first filter reflects the light from the hydrocarbon band and transmits the light for the carbon dioxide and the reference bands. A second filter reflects the light from the reference band and transmits through the light from the carbon dioxide band. FIG. 8 and FIG. 9 show different types of optical coatings that may be used for these two filters. In FIG. 8, reflection percentage **800** goes from a minimum of approximately 3500 nm and then increases up to 3800 nm. Transmissibility **802** has a similar but opposite graph. In FIG. 9, reflection percentage **900** goes from a maximum of approximately 4000 nm and then decreases up to 4150 nm before increasing again at 4600 to return to a high value at 4800. Transmissibility **902** has a similar but opposite graph.

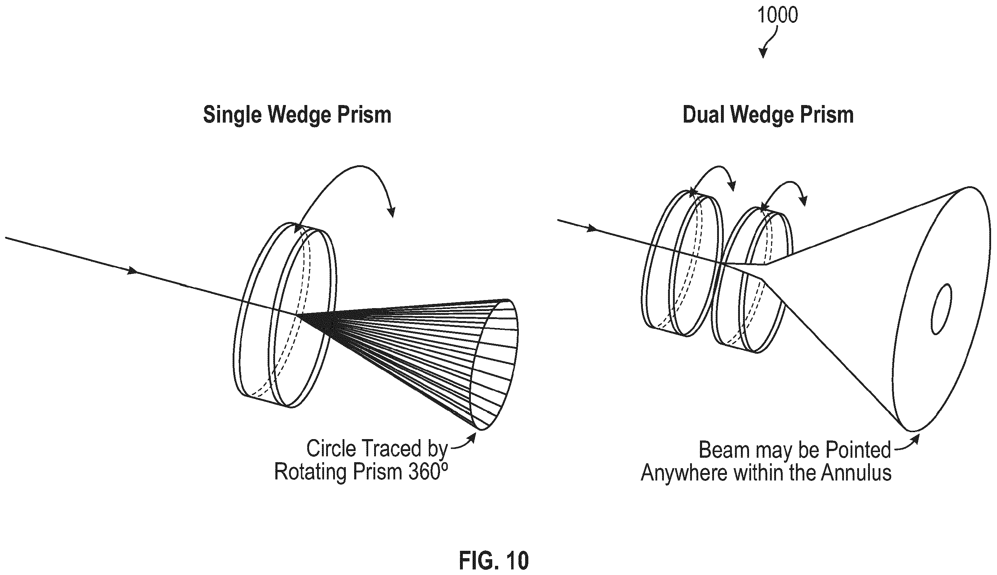

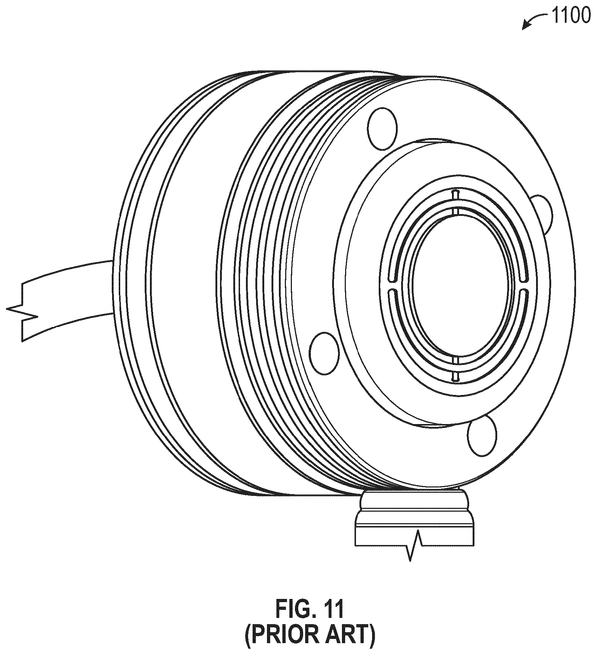

Two different arrangements may be used for the scanner. The first one is based on a dual wedge prism **1000** as shown in FIG. 10. A second solution is to use a fast-steering mirror scanner **1100** as shown in FIG. 11. In further embodiments a galvo scan or a MEMS scan may be used.

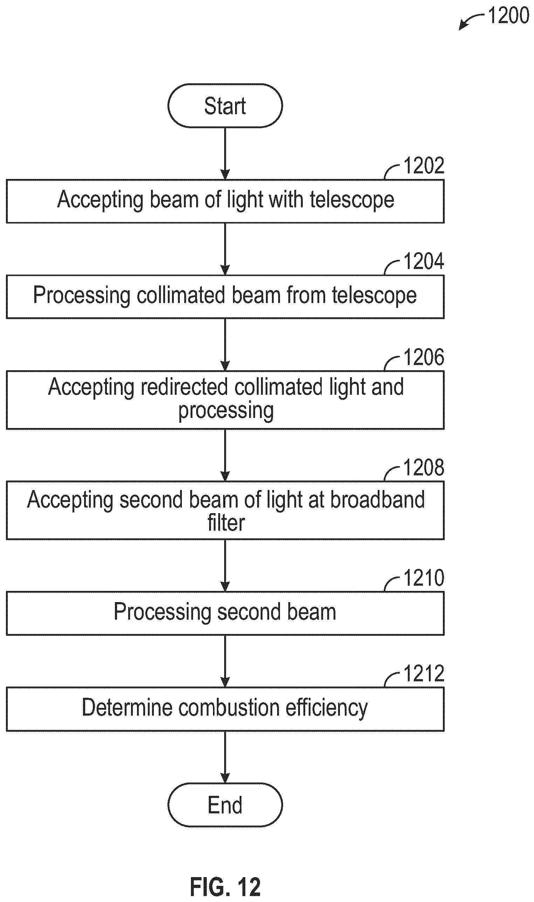

Referring to FIG. 12, a method **1200** is illustrated to determine a combustion efficiency of a combustion source. As will be understood, the combustion source may be a flare or burner as used in industry. The method **1200** may comprise, at **1202,** accepting a beam of light with a telescope from the flare and producing a collimated beam. The method may further comprise, at **1204,** processing the collated beam with a scanner to produce a redirected collimated light. As will be understood, the scanner may use a dual wedge prism or may be a fast-steering mirror scanner. At **1206,** the method may continue with accepting the redirected collimated light at a first lens and processing the redirected collimated light to a second beam. At **1208,** the method may continue with accepting the second beam at a broadband filter and producing a second filtered beam. At **1210,** the method may continue with processing the second filtered beam with a diffraction grating to produce a diffracted collimated light beam. At **1212,** the method may continue with processing the diffracted collimated light beam with a detector array to identify different bands of light to calculate a combustion efficiency. As will be understood, calculating the combustion efficiency may be through use of a computer that will analyze the light bands emanating from the burner or light source and then, based upon the light received, perform simulations of a flare and compare the results of the simulations and actual light bands. Data tables of such types of analysis may be referenced such that a single set of combustion efficiency calculations may be referenced to arrive at results.

The foregoing description of the embodiments has been provided for purposes of illustration and description. It is not intended to be exhaustive or to limit the invention. Individual elements or features of a particular embodiment are generally not limited to that particular embodiment, but, where applicable, are interchangeable and can be used in a selected embodiment, even if not specifically shown or described. The same may be varied in many ways. Such variations are not to be regarded as a departure from the invention, and all such modifications are intended to be included within the scope of the invention.

While embodiments have been described herein, those skilled in the art, having benefit of this invention, will appreciate that other embodiments are envisioned that do not depart from the inventive scope. Accordingly, the scope of the present claims or any subsequent claims shall not be unduly limited by the description of the embodiments described herein.

## Claims

1. An apparatus for analyzing a combustion source, comprising:

    a collimating assembly (305) to accept light from the combustion source and produce a collimated beam,
    a splitting assembly configured to accept the collimated beam and produce at least a first beam, which wavelength is included in a first interest band, and a second beam, which wavelength is included in a second interest band
    a first detector (340) configured to process the first beam,
    a second detector (342) configured to process the second beam,
    wherein the first beam is directed to the first detector (340), and
    wherein the second beam is directed to the second detector (342),

**characterized in that** it comprises a scanning system (307) configured to accept and redirect the collimated beam, the scanning system being configured to aim at different targets.

2. The apparatus of claim 1, wherein the splitting assembly comprises

at least a first beam splitter (302) configured to accept the collimated beam and to split the collimated beam into a first split beam and a second split beam, and
at least a first narrowband filter (320) and a second narrowband filter (322), each configured to accept a split beam and produce a filtered split beam,
wherein the first narrowband filter (320) is configured to accept and filter the first split beam to target the first interest band, and
wherein the first beam is the filtered split beam produced by the first narrower filter (320) and the second beam is the filtered split beam produced by the second narrower filter (322).

3. The apparatus of claim 2, wherein the splitting assembly further comprises

a second beam splitter (304) configured to accept the second split beam and to split the second beam into a third split beam and a fourth split beam, and
a third narrowband filter (324), configured to accept a split beam and produce a filtered split beam,
wherein the second narrowband filter (322) is configured to accept and filter the third split beam to target the second interest band,
wherein the third narrowband filter (324) is configured to accept and filter the fourth split beam to target a third interest band,
wherein a third filtered beam produced by the third narrowband filter (324) forms a third beam produced by the splitting assembly, which wavelength is included in the third interest band, and is directed to a third detector (344) configured to process the third beam.

4. The apparatus of claim 2 or 3, wherein at least one beam splitter (302, 304, 306) is a dichroic mirror.

5. The apparatus of any one of claims 2 to 4, wherein the bandpass width of each narrowband filter (302, 304, 306) is lower or equal to 200 nm.

6. The apparatus of claim 1,

wherein the splitting assembly comprises a diffraction grating (404) configured to accept and process the collimated beam and produce a diffracted collimated light including the first and second beams; and
wherein the first and second detectors are parts of a detector array (402).

7. The apparatus of any one of claims 1 to 6, wherein at least one interest band is a hydrocarbon band, a carbon dioxide band 4190-4330 nm, or a carbon monoxide band.

8. The apparatus of any one claims 1 to 7, wherein the collimating assembly comprises a telescope (305) including a first telescope lens and a second telescope lens, the first telescope lens being configured to accept light from the light source (306) and produce a telescope beam directed to the second telescope lens, the second telescope lens producing the collimated beam.

9. The apparatus of any one of claims 1 to 8, wherein the scanning system (307) uses a dual wedge prism or a fast-steering mirror.

10. The apparatus of any one of claims 1 to 8, wherein the scanning system (307) is a MEMS scanner or a galvo scanner.

11. The apparatus of any one of claims 1 to 10, comprising a broadband filter (312) configured to accept and filter the collimated beam.

12. The apparatus of claim 11, wherein the broadband filter (312) is a dichroic filter.

13. A method for determining a combustion efficiency for a flare, for instance using an apparatus according to any one of the preceding claims, comprising:

accepting light from the flare and producing a collimated beam,
processing the collimated beam to produce at least a first beam, which wavelength is included in a first interest band, and a second beam, which wavelength is included in a second interest band,
processing the first beam and the second beam with a first detector (340) and a second detector (342) to identify different bands of light to calculate a combustion efficiency,
**characterized in that** it comprises aiming at different targets with a scanning system configured to accept and redirect the collimated beam.

**Patentansprüche**

1. Einrichtung zum Analysieren einer Verbrennungsquelle, umfassend:

eine Kollimationsanordnung (305), um Licht von der Verbrennungsquelle anzunehmen und einen kollimierten Strahl zu produzieren, eine Teilungsanordnung, die konfiguriert ist, um den kollimierten Strahl anzunehmen und mindestens einen ersten Strahl, dessen Wellenlänge in einem ersten Interessenband eingeschlossen ist, und einen zweiten Strahl, dessen Wellenlänge in einem zweiten Interessenband eingeschlossen ist, zu produzieren, einen ersten Detektor (340), der konfiguriert ist, um den ersten Strahl zu verarbeiten, einen zweiten Detektor (342), der konfiguriert ist, um den zweiten Strahl zu verarbeiten, wobei der erste Strahl zu dem ersten Detektor (340) geleitet wird und wobei der zweite Strahl zu dem zweiten Detektor (342) geleitet wird, **dadurch gekennzeichnet, dass** sie ein Abtastsystem (307) umfasst, das konfiguriert ist, um den kollimierten Strahl anzunehmen und umzuleiten, wobei das Abtastsystem konfiguriert ist, um unterschiedliche Ziele anzuvisieren.

2. Einrichtung nach Anspruch 1, wobei die Teilungsanordnung umfasst

mindestens einen ersten Strahlteiler (302), der konfiguriert ist, um den kollimierten Strahl anzunehmen und den kollimierten Strahl in einen ersten geteilten Strahl und einen zweiten geteilten Strahl zu teilen, und mindestens einen ersten Schmalbandfilter (320) und einen zweiten Schmalbandfilter (322), die jeweils konfiguriert sind, um einen geteilten Strahl anzunehmen und einen gefilterten geteilten Strahl zu produzieren, wobei der erste Schmalbandfilter (320) konfiguriert ist, um den ersten geteilten Strahl anzunehmen und zu filtern, um das erste Interessenband anzuzielen, und wobei der erste Strahl der gefilterte geteilte Strahl ist, der durch den ersten schmaleren Filter (320) produziert wird, und der zweite Strahl der gefilterte geteilte Strahl ist, der durch den zweiten schmaleren Filter (322) produziert wird.

3. Einrichtung nach Anspruch 2, wobei die Teilungsanordnung ferner umfasst

einen zweiten Strahlteiler (304), der konfiguriert ist, um den zweiten geteilten Strahl anzunehmen und den zweiten Strahl in einen dritten geteilten Strahl und einen vierten geteilten Strahl zu teilen, und einen dritten Schmalbandfilter (324), der konfiguriert ist, um einen geteilten Strahl anzunehmen und einen gefilterten geteilten Strahl zu produzieren, wobei der zweite Schmalbandfilter (322) konfiguriert ist, um den dritten geteilten Strahl anzunehmen und zu filtern, um das zweite Interessenband anzuzielen, wobei der dritte Schmalbandfilter (324) konfiguriert ist, um den vierten geteilten Strahl anzunehmen und zu filtern, um ein drittes Interessenband anzuzielen, wobei ein dritter gefilterter Strahl, der durch den dritten Schmalbandfilter (324) produziert wird, einen dritten Strahl ausbildet, der durch die Teilungsanordnung produziert wird, dessen Wellenlänge in dem dritten Interessenband eingeschlossen ist, und zu einem dritten Detektor (344) geleitet wird, der konfiguriert ist, um den dritten Strahl zu verarbeiten.

4. Einrichtung nach Anspruch 2 oder 3, wobei mindestens ein Strahlteiler (302, 304, 306) ein dichromatischer Spiegel ist.

5. Einrichtung nach einem der Ansprüche 2 bis 4, wobei die Bandpassbreite jedes Schmalbandfilters (302, 304, 306) kleiner oder gleich 200 nm ist.

6. Einrichtung nach Anspruch 1,

wobei die Teilungsanordnung ein Beugungsgitter (404) umfasst, das konfiguriert ist, um den kollimierten Strahl anzunehmen und zu verarbeiten und ein gebeugtes kollimiertes Licht zu produzieren, das den ersten und den zweiten Strahl einschließt; und wobei der erste und der zweite Detektor Bestandteile eines Detektorarrays (402) sind.

7. Einrichtung nach einem der Ansprüche 1 bis 6, wobei mindestens ein Interessenband ein Kohlenwasserstoffband, ein Kohlendioxidband 4190-4330 nm oder ein Kohlenmonoxidband ist.

8. Einrichtung nach einem der Ansprüche 1 bis 7, wobei die Kollimationsanordnung ein Teleskop (305) umfasst, das eine erste Teleskoplinse und eine zweite Teleskoplinse einschließt, wobei die erste Teleskoplinse konfiguriert ist, um Licht von der Lichtquelle (306) anzunehmen und einen Teleskopstrahl zu produzieren, der zu der zweiten Teleskoplinse geleitet wird, wobei die zweite Teleskoplinse den kollimierten Strahl produziert.

9. Einrichtung nach einem der Ansprüche 1 bis 8, wobei das Abtastsystem (307) ein Doppelkeilprisma oder einen schnell steuerbaren Spiegel verwendet.

**10.** Einrichtung nach einem der Ansprüche 1 bis 8, wobei das Abtastsystem (307) ein MEMS-Abtaster oder ein Galvo-Abtaster ist.

**11.** Einrichtung nach einem der Ansprüche 1 bis 10, umfassend einen Breitbandfilter (312), der konfiguriert ist, um den kollimierten Strahl anzunehmen und zu filtern.

**12.** Einrichtung nach Anspruch 11, wobei der Breitbandfilter (312) ein dichromatischer Filter ist.

**13.** Verfahren zum Bestimmen einer Verbrennungseffizienz für eine Fackel, beispielsweise unter Verwendung einer Einrichtung nach einem der vorstehenden Ansprüche, umfassend:

Annehmen von Licht von der Fackel und Produzieren eines kollimierten Strahls,
Verarbeiten des kollimierten Strahls, um mindestens einen ersten Strahl, dessen Wellenlänge in einem ersten Interessenband eingeschlossen ist, und einen zweiten Strahl, dessen Wellenlänge in einem zweiten Interessenband eingeschlossen ist, zu produzieren,
Verarbeiten des ersten Strahls und des zweiten Strahls mit einem ersten Detektor (340) und einem zweiten Detektor (342), um unterschiedliche Lichtbänder zu identifizieren, um eine Verbrennungseffizienz zu berechnen,
**dadurch gekennzeichnet, dass** sie das Anvisieren unterschiedlicher Ziele mit einem Abtastsystem umfasst, das konfiguriert ist, um den kollimierten Strahl anzunehmen und umzuleiten.

**Revendications**

**1.** Appareil destiné à analyser une source de combustion, comprenant :

un ensemble de collimation (305) pour recevoir une lumière à partir de la source de combustion et produire un faisceau collimaté,
un ensemble de division configuré pour recevoir le faisceau collimaté et produire au moins un premier faisceau dont une longueur d'onde est incluse dans une première bande d'intérêt, et un deuxième faisceau dont une longueur d'onde est incluse dans une deuxième bande d'intérêt,
un premier détecteur (340) configuré pour traiter le premier faisceau,
un deuxième détecteur (342) configuré pour traiter le deuxième faisceau,
dans lequel le premier faisceau est dirigé vers le premier détecteur (340), et
dans lequel le deuxième faisceau est dirigé vers le deuxième détecteur (342),
**caractérisé en ce qu'il** comprend un système de balayage (307) configuré pour recevoir et rediriger le faisceau collimaté, le système de balayage étant configuré pour viser différentes cibles.

**2.** Appareil selon la revendication 1, dans lequel l'ensemble de division comprend

au moins un premier diviseur de faisceau (302) configuré pour recevoir le faisceau collimaté et pour diviser le faisceau collimaté en un premier faisceau divisé et un deuxième faisceau divisé, et
au moins un premier filtre à bande étroite (320) et un deuxième filtre à bande étroite (322), chacun étant configuré pour recevoir un faisceau divisé et produire un faisceau divisé filtré,
dans lequel le premier filtre à bande étroite (320) est configuré pour recevoir et filtrer le premier faisceau divisé afin de cibler la première bande d'intérêt, et
dans lequel le premier faisceau est le faisceau divisé filtré produit par le premier filtre plus étroit (320) et le deuxième faisceau est le faisceau divisé filtré produit par le deuxième filtre plus étroit (322).

**3.** Appareil selon la revendication 2, dans lequel l'ensemble de division comprend en outre

un deuxième diviseur de faisceau (304) configuré pour recevoir le deuxième faisceau divisé et pour diviser le deuxième faisceau en un troisième faisceau divisé et un quatrième faisceau divisé, et
un troisième filtre à bande étroite (324), configuré pour recevoir un faisceau divisé et produire un faisceau divisé filtré,
dans lequel le deuxième filtre à bande étroite (322) est configuré pour recevoir et filtrer le troisième faisceau divisé afin de cibler la deuxième bande d'intérêt,
dans lequel le troisième filtre à bande étroite (324) est configuré pour recevoir et filtrer le quatrième faisceau divisé afin de cibler une troisième bande d'intérêt,
dans lequel un troisième faisceau filtré produit par le troisième filtre à bande étroite (324) forme un troisième faisceau produit par l'ensemble de division, dont une longueur d'onde est incluse dans la troisième bande d'intérêt, et est dirigé vers un troisième détecteur (344) configuré pour traiter le troisième faisceau.

**4.** Appareil selon la revendication 2 ou 3, dans lequel au moins un diviseur de faisceau (302, 304, 306) est un miroir dichroïque.

5. Appareil selon l'une quelconque des revendications 2 à 4, dans lequel la largeur de bande passante de chaque filtre à bande étroite (302, 304, 306) est inférieure ou égale à 200 nm.

6. Appareil selon la revendication 1,

dans lequel l'ensemble de division comprend un réseau de diffraction (404) configuré pour recevoir et traiter le faisceau collimaté et produire une lumière collimatée diffractée comportant les premier et deuxième faisceaux ; et
dans lequel les premier et deuxième détecteurs font partie d'un réseau de détecteurs (402).

7. Appareil selon l'une quelconque des revendications 1 à 6, dans lequel au moins une bande d'intérêt est une bande d'hydrocarbures, une bande de dioxyde de carbone de 4 190 à 4 330 nm, ou une bande de monoxyde de carbone.

8. Appareil selon l'une quelconque des revendications 1 à 7, dans lequel l'ensemble de collimation comprend un télescope (305) comportant une première lentille de télescope et une seconde lentille de télescope, la première lentille de télescope étant configurée pour recevoir une lumière à partir de la source de lumière (306) et produire un faisceau de télescope dirigé vers la seconde lentille de télescope, la seconde lentille de télescope produisant le faisceau collimaté.

9. Appareil selon l'une quelconque des revendications 1 à 8, dans lequel le système de balayage (307) utilise un prisme à double coin ou un miroir à orientation rapide.

10. Appareil selon l'une quelconque des revendications 1 à 8, dans lequel le système de balayage (307) est un scanner MEMS ou un scanner galvo.

11. Appareil selon l'une quelconque des revendications 1 à 10, comprenant un filtre à bande large (312) configuré pour recevoir et filtrer le faisceau collimaté.

12. Appareil selon la revendication 11, dans lequel le filtre à bande large (312) est un filtre dichroïque.

13. Procédé destiné à déterminer un rendement de combustion pour une torche, par exemple à l'aide d'un appareil selon l'une quelconque des revendications précédentes, comprenant :

la réception de lumière à partir de la torche et la production d'un faisceau collimaté,
le traitement du faisceau collimaté pour produire au moins un premier faisceau dont une longueur d'onde est incluse dans une première bande d'intérêt, et un deuxième faisceau dont une longueur d'onde est incluse dans une deuxième bande d'intérêt,
le traitement du premier faisceau et du deuxième faisceau avec un premier détecteur (340) et un deuxième détecteur (342) pour identifier différentes bandes de lumière afin de calculer un rendement de combustion,
**caractérisé en ce qu'il** comprend le fait de viser différentes cibles avec un système de balayage configuré pour recevoir et rediriger le faisceau collimaté.

Samples for CE
Calculation

FIG. 1

**FIG. 2**

CO2

CO

4190 - 4330nm

REF

3855 - 3945nm

CH4

3220 - 3380nm

5.0µm

2.5µm

FIG. 3

EP 4 502 469 B1

FIG. 4

EP 4 502 469 B1

FIG. 5
(PRIOR ART)

Band 2 (reflected)

Incident: Radiation
Band 1 and Band 2

Band 1 (transmitted)

Dichroic Filter at
45 Degrees AOI

FIG. 6

EP 4 502 469 B1

3200-3400 →

3800-4400 →

$\uparrow$ 3200 -3400

DF1 / 45 Degrees

3800-4400 →

$\uparrow$ 3800 -4000

DF2 /

4150-4400 →

**FIG. 7**

FIG. 8

FIG. 9

FIG. 10

1100

FIG. 11
(PRIOR ART)

1200

```
        ┌─────────────┐
        │    Start    │
        └─────────────┘
               │
               ▼                              ⌐1202
   ┌──────────────────────────────────────┐
   │  Accepting beam of light with telescope │
   └──────────────────────────────────────┘
               │
               ▼                              ⌐1204
   ┌──────────────────────────────────────┐
   │  Processing collimated beam from telescope │
   └──────────────────────────────────────┘
               │
               ▼                              ⌐1206
   ┌──────────────────────────────────────┐
   │   Accepting redirected collimated light and │
   │              processing                │
   └──────────────────────────────────────┘
               │
               ▼                              ⌐1208
   ┌──────────────────────────────────────┐
   │  Accepting second beam of light at broadband │
   │                filter                  │
   └──────────────────────────────────────┘
               │
               ▼                              ⌐1210
   ┌──────────────────────────────────────┐
   │        Processing second beam          │
   └──────────────────────────────────────┘
               │
               ▼                              ⌐1212
   ┌──────────────────────────────────────┐
   │     Determine combustion efficiency     │
   └──────────────────────────────────────┘
               │
               ▼
        ┌─────────────┐
        │     End     │
        └─────────────┘
```

FIG. 12

**REFERENCES CITED IN THE DESCRIPTION**

**Patent documents cited in the description**

- US 20010035952 A
- WO 02070953 A
- WO 2021085716 A
- CN 101625270